(19) Europäisches Patentamt / European Patent Office / Office européen des brevets

(11) **EP 3 725 285 A1**

(12) **EUROPEAN PATENT APPLICATION**
published in accordance with Art. 153(4) EPC

(43) Date of publication:
**21.10.2020 Bulletin 2020/43**

(51) Int Cl.:
**A61J 1/05** (2006.01)

(21) Application number: **18887773.2**

(22) Date of filing: **13.12.2018**

(86) International application number:
**PCT/JP2018/045986**

(87) International publication number:
**WO 2019/117267 (20.06.2019 Gazette 2019/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.12.2017 JP 2017240315**
**12.12.2018 JP 2018232949**

(71) Applicant: **Nipro Corporation**
**Osaka-shi, Osaka 531-8510 (JP)**

(72) Inventors:
• **SUZUKI, Tetsuya**
**Yokohama-shi, Kanagawa 224-0033 (JP)**
• **SHIRAKURA, Akira**
**Tokyo 150-0031 (JP)**
• **OOKAWA, Hiromasa**
**Osaka-shi, Osaka 531-8510 (JP)**
• **MEKATA, Yoshimi**
**Osaka-shi, Osaka 531-8510 (JP)**

(74) Representative: **v. Bezold & Partner Patentanwälte
- PartG mbB**
**Akademiestraße 7**
**80799 München (DE)**

(54) **MEDICAL GLASS CONTAINER AND METHOD FOR MANUFACTURING SAME**

(57) The present disclosure provides a medical glass container and a method for manufacturing the same, in which a contact angle with an aqueous content is large, transparency is high, lubricity (slidability) is excellent, peeling of a silica ($SiO_2$) component of glass, which is a base material of the container, does not easily occur, aggregation (absorption) of protein, which is an effective component of a medicine, does not easily occur, it is heat resistant, and peeling of a coat is suppressed. In the medical glass container according to the present disclosure, a coat is formed on at least a part of an inner wall of the glass container, wherein the coat is a diamond-like carbon film that does not contain silicon.

[FIG.1]

**Description**

TECHNICAL FIELD

The present disclosure relates to a medical glass container, and more particularly, to a glass container for storing and preserving a medicine for medical purposes. The medical glass container includes, for example, a vial, an injector barrel (syringe), a syringe with needle, and a cartridge type syringe.

BACKGROUND ART

A medical glass container that contains silicon, oxygen, carbon, hydrogen, and fluorine on the inner surface of the container and has an anti-adhesion coating formed by a plasma-enhanced chemical vapor deposition process has been disclosed (for example, see Patent Document 1). Patent Document 1 discloses that this anti-adhesion coating is amorphous and transparent, satisfies the pharmaceutical requirements, and has a wet contact angle with water of 80° or greater.

A technique of applying a coating material having lubricity to a container by using PECVD (plasma-enhanced chemical vapor deposition) has been disclosed (for example, see Patent Document 2). Patent Document 2 discloses that this coating contains silicon, oxygen, carbon, and hydrogen, and is a low friction coating material for a plastic syringe.

In both the techniques disclosed in Patent Documents 1 and 2, it is disclosed that the silicon oxide-based thin film does not sufficiently suppress protein adsorption while the container is coated with a silicon oxide-based thin film (for example, see Non-Patent Document 1).

Patent Document 1: Japanese Unexamined Patent Application Publication No. 2000-350770
Patent Document 2: Japanese Unexamined Patent Application Publication No. 2015-180780
Non-Patent Document 1: Journal of Colloid and Interface Science, Vol. 166, Issue 2, September 1994, 490-498, Martinmalmosten, Bo Lassen

DISCLOSURE OF THE INVENTION

PROBLEMS TO BE SOLVED BY THE INVENTION

In the technique disclosed in Patent Document 1, since the container has a water-repellent property, residual chemicals is easily removed. However, the anti-adhesion coating is a silicon-containing coat, and, considering Non-Patent Document 1, a glass container coated with a material having a composition different from that of this coat, that is, a glass container having a coat mainly consisting of carbon and hydrogen close to living-body components, is particularly desirable as biological agents containing living-body-derived substances have become popular in recent years.

In addition, in the technique disclosed in Patent Document 2, the silicon-containing coating material is not suitable for a glass syringe because it is formed for the purposes of improving lubricity, and its target is a plastic syringe. That is, the technique of Patent Document 2 is merely intended to reduce an extruding force of a plunger having a rubber gasket of a plastic syringe to the same level as that of the glass syringe. As prefilled syringes have become popular in recent years, a gasket having a high sealing property without leakage for a long period of time is demanded, and it is required to reduce the extruding force of the plunger in the glass syringe.

In this regard, an object of the present disclosure is to provide a medical glass container, in which a contact angle with an aqueous content is large, transparency is high, lubricity (slidability) is excellent, peeling of the silica component ($SiO_2$) of the glass, which is a base material of the container, does not easily occur, elution of a glass-derived component

(silicon, boron, sodium, potassium, or aluminum) into the content is negligible, aggregation (absorption) of protein, which is an effective component of a medicine, does not easily occur, there is heat resistance, and peeling of the coat is suppressed. Particularly, the object is to provide a medical glass container and a method for manufacturing the same, in which at least a part of a surface of the container is coated with a silicon-free diamond-like carbon film.

SOLUTIONS TO THE PROBLEMS

The inventors made diligent studies and completed the present invention by finding that the aforementioned problems are solved by coating at least a part of a surface of a glass container such as an inner surface thereof with a silicon-free diamond-like carbon film. That is, a medical glass container according to the present invention has a coat formed on at least a part of an inner wall of a glass container, wherein the coat is a silicon-free diamond-like carbon film.

In the medical glass container according to the present invention, the coat includes a silicon-free and fluorine-containing diamond-like carbon film or a silicon-free and fluorine-free diamond-like carbon film.

In the medical glass container according to the present invention, it is preferable that a silicon-containing intermediate layer is provided between a glass surface of the inner wall of the glass container and the coat. Since the silicon-containing intermediate layer is provided, adhesion of the coat is improved, and transparency and heat resistance are improved.

In the medical glass container according to the present invention, it is preferable that at least a part of the glass surface of the inner wall of the glass container is hydrophilized. Since the glass surface of the inner wall of the glass container is hydrophilized, adhesion of the coat is improved.

In the medical glass container according to the present invention, glass forming the glass container includes borosilicate glass, aluminosilicate glass, or soda lime glass.

In the medical glass container according to the present invention, the glass container includes a vial, an injector barrel, a syringe with needle, and a cartridge type syringe.

In the medical glass container according to the present invention, it is preferable that the glass container is a vial, the medical glass container further has a rubber stopper, and at least a part of an inner surface of the rubber stopper is coated with a diamond-like carbon film or a fluororesin film. The content contained in the container does not come into contact with glass or rubber, but can come into contact with only the diamond-like carbon film or only both the diamond-like carbon film and the fluororesin film, so that stability of the content is improved.

In the medical glass container according to the present invention, it is preferable that the glass container is an injector barrel, the medical glass container further has a gasket-mounted plunger, and at least a part of a surface of the gasket is coated with a diamond-like carbon film. It is possible to smoothly move the plunger when it is pushed.

In the medical glass container according to the present invention, it is preferable that the glass container is an injector barrel, the medical glass container further has a gasket-mounted plunger, and a fluororesin film is bonded to at least a face of a surface of the gasket adjoining with the inner surface of the injector barrel. It is possible to smoothly move the plunger when it is pushed.

In the medical glass container according to the present invention, it is preferable that the coat is a silicon-free and fluorine-containing diamond-like carbon film, and a transmittance of the glass container at 450 nm is 90 % or higher. It is possible to visually clearly recognize the content.

According to the present invention, there is provided a method for manufacturing a medical glass container having a coat formed on at least a part of an inner wall of the glass container, wherein the method has a process of forming a silicon-free diamond-like carbon film as the coat by plasmatizing a hydrocarbon-based gas that does not contain silicon as a constituent element inside a storage space of the glass container.

In the method for manufacturing the medical glass container according to the present invention, it is preferable that the hydrocarbon-based gas that does not contain silicon is a mixed gas of a first hydrocarbon-based gas that does not contain fluorine or silicon as a constituent element and a second hydrocarbon-based gas that is modified with fluorine and does not contain silicon as a constituent element, and the coat is a silicon-free/fluorine-containing diamond-like carbon film. Using the aforementioned mixed gas, it is possible to easily set a desired ratio of the fluorine content in the coat.

In the method for manufacturing the medical glass container according to the present invention, it is preferable that the hydrocarbon-based gas that does not contain silicon is a first hydrocarbon-based gas that does not contain fluorine or silicon as a constituent element, and the coat is a silicon-free/fluorine-containing diamond-like carbon film. It is possible to easily form the silicon-free/fluorine-free diamond-like carbon film.

In the method for manufacturing the medical glass container according to the present invention, it is preferable that the first hydrocarbon-based gas is acetylene and/or methane. Using the aforementioned source gas as the first hydrocarbon-based gas, it is possible to obtain a diamond-like carbon film having little coloring and excellent heat resistance.

In the method for manufacturing the medical glass container according to the present invention, it is preferable that a gas volume ratio of the mixed gas satisfies a range of Formula 1. Since the fluorine content of the coat becomes within a predetermined range, for example, 8 to 50 atomic% (atomic percentage), an excellent water repellency effect is obtained.

(Formula 1)

(second hydrocarbon-based gas):(first hydrocarbon-based gas) = 7:3 to 9:1.

In the method for manufacturing the medical glass container according to the present invention, it is preferable that the hydrocarbon-based gas that does not contain silicon is plasmatized by high frequency output or microwave output. In this configuration, it is possible to easily plasmatize the source gas and form a coat having a high density on the inner surface of the container.

In the method for manufacturing the medical glass container according to the present invention, it is preferable that the method further has a process of forming an intermediate layer on a glass surface of the inner wall of the glass container by plasmatizing a silicon-containing gas inside a storage space of the glass container before forming the silicon-free diamond-like carbon film as the coat. By providing the silicon-containing intermediate layer, adhesion of the coat is improved.

In the method for manufacturing the medical glass container according to the present invention, it is preferable that the method further has a hydrophilizing process for forming plasma by bringing a hydrocarbon-based gas or an oxygen gas modified with fluorine into contact with at least a part of an inner surface of the medical glass container before forming the coat and/or the intermediate layer. Since the glass surface of the inner wall of the glass container is hydrophilized, adhesion of the coat is improved, and transparency and heat resistance are improved.

EFFECTS OF THE INVENTION

According to the present disclosure, it is possible to provide a medical glass container, in which a contact angle with an aqueous content is large, transparency is high, lubricity (slidability) is excellent, peeling (delamination) of the silica component of the glass, which is a base material of the container, does not easily occur, elution of a glass-derived component (silicon, boron, sodium, potassium, or aluminum) into the content is negligible, aggregation (absorption) of protein, which is an effective component of a medicine, does not easily occur, there is heat resistance, and peeling of the coat is suppressed. Particularly, it is possible to provide a medical glass container and a method for manufacturing the same, in which at least a part of the surface of the container is coated with a silicon-free diamond-like carbon film.

BRIEF DESCRIPTION OF THE DRAWINGS

FIG. 1 is a cross-sectional view when a medical glass container according to the present embodiment is a vial.
FIG. 2 is a cross-sectional view when the medical glass container according to the present embodiment is an injector barrel.
FIG. 3 is a schematic diagram illustrating a high frequency type inner surface film formation apparatus for a vial.
FIG. 4 is a schematic diagram illustrating a microwave type inner surface film formation apparatus for a vial.
FIG. 5 is a schematic diagram illustrating a high frequency type inner surface film formation apparatus for an injector barrel.
FIG. 6 is a schematic diagram illustrating a microwave type inner surface film formation apparatus for an injector barrel.

DESCRIPTION OF EMBODIMENTS

While embodiments of the present invention will now be described in details, the present invention is not construed as being limited to such descriptions. Various modifications may be possible as long as the effects of the present invention are exhibited.

The medical glass container according to the present embodiment has a coat formed on at least a part of the inner wall of the glass container, wherein the coat is a silicon-free diamond-like carbon film.

(Glass Container)

The medical glass container includes a vial, an injector barrel, a syringe with needle, and a cartridge type syringe. Note that the injector barrel includes a syringe with needle and a cartridge type syringe. Glass forming the glass container includes borosilicate glass, aluminosilicate glass, or soda lime glass.

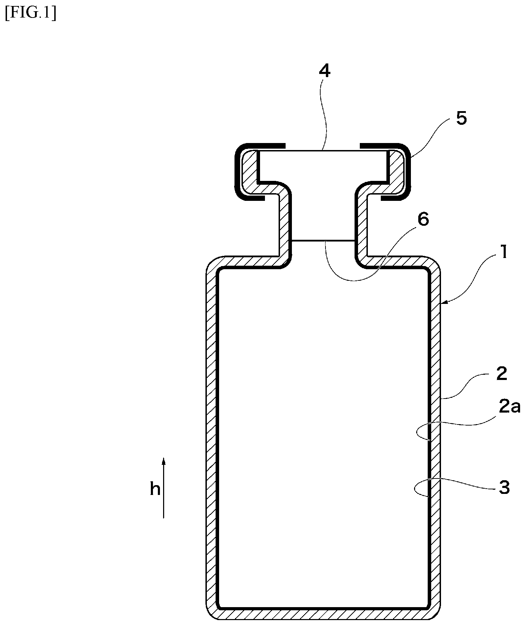

As illustrated in FIG. 1, when the medical glass container 1 is in the form of a vial, a coat 3 is formed on an inner wall 2a of the vial 2 at an area ratio of preferably 15% or higher, more preferably 30% or higher, and still more preferably 80% or higher. It is preferable that the coat 3 is formed on the entire bottom surface of the storage space of the vial. In addition, it is more preferable that the inner wall surface of the container lower than the center in height direction of the container h is formed on the entire surface. Furthermore, it is particularly preferable that the coat 3 is formed on the entire surface of the inner wall 2a of the glass container (vial 2). Peeling (delamination) of the silica component ($SiO_2$ component) of the glass, which is a base material of the vial 2, does not easily occur due to presence of the coat 3. In particular, when the vial is formed of borosilicate glass, peeling of silica tends to easily occur. However, this peeling can be suppressed.

In addition, in the medical glass container 1 according to the present embodiment, the glass container is the vial 2 and further has a rubber stopper 4, and at least a part of the inner surface of the rubber stopper 4 is preferably coated with a diamond-like carbon film 6 or a fluororesin film. The rubber stopper 4 is formed of thermoplastic elastomer, butyl rubber (isobutyl/isoprene rubber), silicone rubber, or the like. Since the medicine contained in the vial 2 can be brought into contact with only the diamond-like carbon film or with only both the diamond-like carbon film and the fluororesin film without contact with glass or rubber, stability of the content is improved.

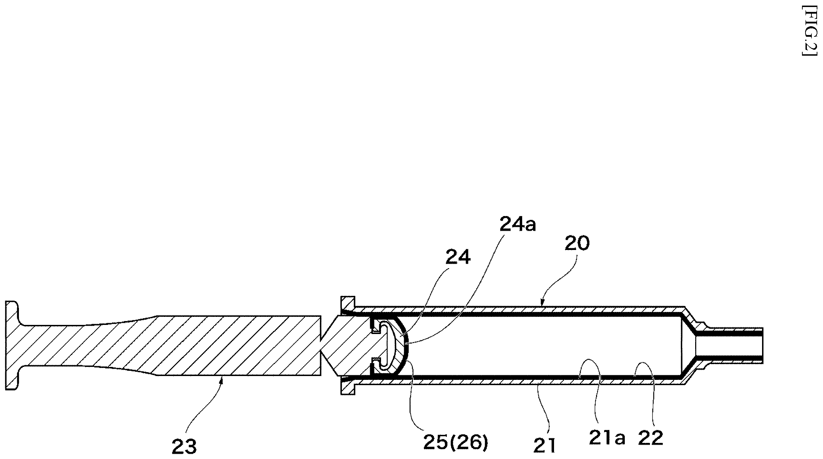

As illustrated in FIG. 2, when the medical glass container 20 is in the form of injector barrel (syringe), a coat 22 is preferably formed on a surface where at least the glass container (injector barrel 21) and a gasket 24 installed in a plunger 23 can be brought into contact by sliding. Due to the presence of the coat 22, it is possible to improve lubricity, that is, slidability of the plunger 23, so that the plunger 23 can be smoothly moved when it is pushed. Furthermore, the coat 22 is preferably formed on the entire surface of the inner wall 21a of the injector barrel 21. In addition to improvement of slidability, the coat 22 prevents contact between a medicinal liquid which is the content and glass which is a constituent material of the injector barrel 21, and due to the coat 22 having high water-repellent property, residual liquid is reduced.

In the present embodiment, the injector barrel 21 may be a prefilled syringe as well as a normal syringe. In addition, the shape of the tip of the injector barrel is not particularly limited, and various shapes such as a luer slip type, a luer lock type, a luer metal type, a catheter tip type, and an enema type may be employed.

As illustrated in FIG. 2, in the medical glass container according to the present embodiment, the medical glass container 20 is an injector barrel, and further includes the plunger 23 in which the gasket 24 is installed, and at least a part of the surface 24a of the gasket 24 is preferably coated with the diamond-like carbon film 25. The diamond-like carbon film 25 is preferably formed on a portion of the surface of the gasket 24 that comes into contact with the inner surface 21a of the injector barrel 21. The diamond-like carbon film 25 formed on the surface of the gasket 24 and the inner surface of the glass container 20 (surface of the coat 22) come into contact with each other, so that the plunger 23 can be moved more smoothly when it is pushed. In addition, the diamond-like carbon film 25 is preferably formed on a portion of the surface of the gasket 24 that comes into contact with the medicine. In addition to improvement of slidability of the plunger 23, it is possible to prevent contact between the gasket 24 and the medicine. Furthermore, more preferably, the diamond-like carbon film 25 is formed on both a portion of the surface of the gasket 24 that comes into contact with the inner surface of the injector barrel and a portion that comes into contact with the medicine. Note that the gasket 24 is formed of thermoplastic elastomer (such as butyl rubber or isoprene rubber), plastic (such as polypropylene or polystyrene), or the like.

A fluororesin film 26 may be arranged instead of the diamond-like carbon film 25 formed on the surface of the gasket 24 illustrated in FIG. 2. That is, in the medical glass container according to the present embodiment, it is preferable that the medical glass container 20 is an injector barrel, and further has the plunger 23 in which the gasket 24 is installed, and a fluororesin film 26 is bonded to at least a part of the surface 24a of the gasket 24 adjoining with the inner surface of the medical glass container 20 (the surface of the coat 22). It is possible to more smoothly move the plunger 23 when it is pushed. The fluororesin film may be formed of one or more fluororesins such as polytetrafluoroethylene, tetrafluoroethylene/perfluoroalkyl vinyl ether copolymer, tetrafluoroethylene/hexafluoropropylene copolymer, tetrafluoroethylene/ethylene copolymer, polyvinylidene fluoride, polychlorotrifluoroethylene, and chlorotrifluoroethylene/ethylene copolymer.

(Coat)

The coats 3 and 22 are silicon-free diamond-like carbon films. Here, the diamond-like carbon is also called a diamond-like carbon film, a DLC film, or an amorphous carbon film, and is a hydrogenated amorphous carbon film containing at least a carbon atom and a hydrogen atom. The coats 3 and 22 preferably have a thickness of 1 to 70 nm, and more preferably 2 to 10 nm. If the thickness is less than 1 nm, it may be difficult to form a uniform film without a defect. If the thickness exceeds 70 nm, peeling may occur, or coloring may exceed an allowable range.

Here, the coat 3 or 22 as a silicon-free diamond-like carbon film is a silicon-free and fluorine-containing diamond-like carbon film (hereinafter, referred to as "F-DLC film" in some cases) or a silicon-free and fluorine-free diamond-like carbon film (hereinafter, simply referred to as "DLC film" in some cases). Note that the fluorine-containing diamond-like carbon film is also called a fluorinated amorphous carbon film.

When the coat 3 or 22 is a silicon-free and fluorine-free diamond-like carbon film, the water-repellent property, the heat resistance, and the protein non-adsorption property are excellent.

When the coat 3 or 22 is a silicon-free and fluorine-containing diamond-like carbon film, the transparency, water-repellent property, the protein non-adsorption property, and the heat resistance are excellent. The fluorine content in the coat 3 or 22 is preferably 10 to 50 atomic% on the outermost surface of the coat. If the fluorine content is less than 10 atomic%, the act of the fluorine becomes less significant, and, for example, the water-repellent property and the transparency may decrease. If the fluorine content is more than 50 atomic%, the heat resistance may decrease. The fluorine content in the coat 3 or 22 is preferably in the range of 0 to 20 atomic% on the innermost surface of the coat in order to maintain adhesion. For this reason, the fluorine content preferably has a gradient composition that increases from the inner surface to the outermost surface of the coat.

According to the present embodiment, a silicon-containing intermediate layer is preferably provided between the glass surface of the inner wall and the coat of the glass container 2 or 21. By providing the silicon-containing intermediate layer, the adhesion of the film is improved. Specifically, there are (1) a mode in which a silicon-containing intermediate layer is formed on the glass surface of the inner wall of the glass container 2 or 21, and a silicon-free and fluorine-free diamond-like carbon film is further formed thereon, and (2) a mode in which a silicon-containing intermediate layer is formed on the glass surface of the inner wall of the glass container 2 or 21, and a silicon-free and fluorine-containing diamond-like carbon film is further formed thereon.

The mode in which the silicon-containing intermediate layer is formed on the glass surface of the inner wall of the glass container 2 or 21, and the silicon-free and fluorine-free diamond-like carbon film is further formed thereon will be described. It is preferable that the silicon-containing intermediate layer is, for example, a silicon oxide film, and the silicon oxide film contains silicon, oxygen, carbon, and hydrogen, and has a thickness of 3 to 50 nm. The thickness of the silicon oxide film is more preferably 5 to 20 nm. If the thickness of the silicon oxide film is smaller than 3 nm, the act of the intermediate layer becomes less significant, and the adhesion of the coat provided on the intermediate layer may decrease. If the thickness of the silicon oxide film is larger than 50 nm, a crack may be generated due to internal stress, or it may take time for film formation. In addition, the silicon-free and fluorine-free diamond-like carbon film provided on the intermediate layer preferably has a density of 1.6 to 2.4 $g/cm^3$, a hydrogen content of 8 to 40 atomic%, and a thickness of 1 to 70 nm. Here, the density is more preferably 1.8 to 2.2 $g/cm^3$, the hydrogen content is more preferably 10 to 30 atomic%, and the thickness is more preferably 2 to 10 nm. If the density is lower than 1.6 $g/cm^3$, the heat resistance may decrease. If the density is higher than 2.4 $g/cm^3$, a crack may be generated, or coloring may become severe. If the hydrogen content is less than 8 atomic%, the density may increase, which may cause cracking or severe coloring. If the hydrogen content is more than 40 atomic%, the density may decrease, and the heat resistance may decrease. If the thickness is smaller than 1 nm, it may be difficult to uniformly form the film, and a defect may be generated in the coat. If the thickness is larger than 70 nm, the transparency may decrease.

The mode in which the silicon-containing intermediate layer is formed on the glass surface of the inner wall of the glass container 2 or 21, and the silicon-free and fluorine-containing diamond-like carbon film is further formed thereon will be described. It is preferable that the silicon-containing intermediate layer is, for example, a silicon oxide film, and the silicon oxide film contains silicon, oxygen, carbon, and hydrogen, and may contain fluorine in some cases, and the thickness is 3 to 50 nm. The thickness of the silicon oxide film is more preferably 5 to 20 nm. If the thickness of the silicon oxide film is smaller than 3 nm, the act of the intermediate layer becomes less significant, so that the adhesion of the coat provided on the intermediate layer may decrease. If the thickness of the silicon oxide film is larger than 50 nm, a crack may be generated due to internal stress, or it may take time for film formation. In addition, it is preferable that the silicon-free and fluorine-containing diamond-like carbon film provided on the intermediate layer has a density of 1.6 to 2.4 $g/cm^3$, a hydrogen content of 8 to 40 atomic%, an average fluorine content in the film of 10 to 20 atomic%, a fluorine content on the outermost surface of 8 to 50 atomic%, and a thickness of 1 to 70 nm. Here, more preferably, the density is 1.8 to 2.2 $g/cm^3$, the hydrogen content is 10 to 30 atomic%, the fluorine content on the outermost surface is 10 to 40 atomic%, and the thickness is 2 to 10 nm. If the density is lower than 1.6 $g/cm^3$, the heat resistance may decrease. If the hydrogen content is less than 8 atomic%, the density may increase, a crack may be generated, or coloring may become severe. If the hydrogen content is more than 40 atomic%, the density may decrease, and the heat resistance may decrease. If the fluorine content is less than 8 atomic%, the act of the fluorine becomes less significant, and, for example, the water-repellent property and the transparency may decrease. If the fluorine content is more than 50 atomic%, the heat resistance may decrease. If the thickness is smaller than 1 nm, uniformity of film formation may be degraded. If the thickness is larger than 70 nm, peeling may occur, or coloring may exceed an allowable range.

In the mode in which the silicon-containing intermediate layer is formed, and the silicon-free and fluorine-containing diamond-like carbon film is further formed thereon, the intermediate layer and the silicon-free and fluorine-containing diamond-like carbon film may have a gradient composition, so that they may form a substantially single-layered gradient composition film. Specifically, in the coats 3 and 22, (1) the outermost surface is a silicon-free and fluorine-containing diamond-like carbon surface, and the surface adjoining with the container inner surface is a silicon oxide surface or a fluorine-containing silicon oxide surface. (2) The gradient composition film has a density of 1.6 to 2.4 $g/cm^3$ or preferably 1.8 to 2.2 $g/cm^3$ from the outermost surface to a thickness of 1 nm. In addition, the hydrogen content from the outermost surface to the thickness of 1 nm is 8 to 40 atomic% or preferably 10 to 30 atomic%. In addition, the fluorine content from the outermost surface to the thickness of 1 nm is 8 to 50 atomic% or preferably 10 to 40 atomic%. (3) The surface of the silicon oxide adjoining with the inner surface of the container may contain silicon, oxygen, carbon, and hydrogen, and may contain fluorine. (4) The composition gradually changes in the thickness direction of the coat from the surface adjoining with the inner surface of the container to the outermost surface, and the thickness of the coat 3 or 22 as the gradient composition film is 1 to 70 nm or preferably 5 to 20 nm. If the density in the description (2) is less than 1.6 $g/cm^3$, the heat resistance may decrease. If the density is higher than 2.4 $g/cm^3$, a crack may occur, or coloring may become severe. If the hydrogen content is less than 8 atomic%, the density may decrease, and the heat resistance may decrease. If the hydrogen content is more than 40 atomic%, the density may increase, a crack may be generated, or coloring may become severe. If the fluorine content is less than 8 atomic%, the act of fluorine becomes less significant, and for example, the water-repellent property and the transparency may decrease. If the fluorine content is more than 50 atomic%, the heat resistance may decrease. If the thickness in the description (4) is smaller than 1 nm, uniformity of film formation may be degraded. If the thickness is larger than 70 nm, peeling may occur, or coloring may exceed an allowable range.

In the medical glass container according to the present embodiment, at least a part of the glass surface of the inner wall of the glass container 2 or 21 is preferably hydrophilized in the mode in which the intermediate layer is provided. By hydrophilizing the glass surface of the inner wall of the glass container 2 or 21, adhesion of the coat including the intermediate film is improved. In the hydrophilizing treatment, for example, a carbonyl group or a carboxyl group may be added to the glass surface, or an OH group may be added or bonded. When the medical glass container 1 or 20 is employed, a layer containing a carbonyl group with a thickness of 0.5 to 10 nm is preferably formed at the interface between the intermediate layer and the inner surface of the container, and more preferably, a layer containing a carbonyl group with a thickness of 1 to 5 nm is formed. In the hydrophilized form, the silicon-containing intermediate layer is, for example, a silicon oxide film, and the silicon oxide film contains silicon, oxygen, carbon, and hydrogen, and may contain fluorine. The film thickness is more preferably 3 to 50 nm. The thickness of the silicon oxide film is more preferably 5 to 20 nm. If the thickness of the silicon oxide film is smaller than 3 nm, the act of the intermediate layer becomes less significant, and adhesion of the coat provided on the intermediate layer may decrease. If the thickness of the silicon oxide film is larger than 50 nm, a crack may be generated due to internal stress, and it may take time for film formation.

The diamond-like carbon film 6 formed on the surface of the rubber stopper 4 and the diamond-like carbon film 25 formed on the surface of the gasket 24 preferably contain hydrogen of 30 to 45 atomic% and fluorine of 0 to 20 atomic%, and has a thickness of preferably 1 to 70 nm or more preferably 5 to 20 nm.

The fluororesin film that coats the surface of the rubber stopper 4 may be formed of one or more fluororesins such as polytetrafluoroethylene, tetrafluoroethylene/perfluoroalkyl vinyl ether copolymer, tetrafluoroethylene/hexafluoropropylene copolymer, tetrafluoroethylene/ethylene copolymer, polyvinylidene fluoride, polychlorotrifluoroethylene, or chlorotrifluoroethylene/ethylene copolymer.

The medical glass container 1 or 20 preferably has a light-transmitting property. Specifically, the transmittance at a wavelength of 590 to 610 nm or 290 to 450 nm is preferably 45% or higher, or more preferably 60% or higher. An evaluation method for a transparency test is based on "Japanese Pharmacopoeia (17th Edition), 7. Tests for Containers and Packing Materials, 7.01 Test for Glass Containers for Injections, (5) Light transmission test for light-resistant containers".

(Apparatus for Manufacturing Medical Glass Container)

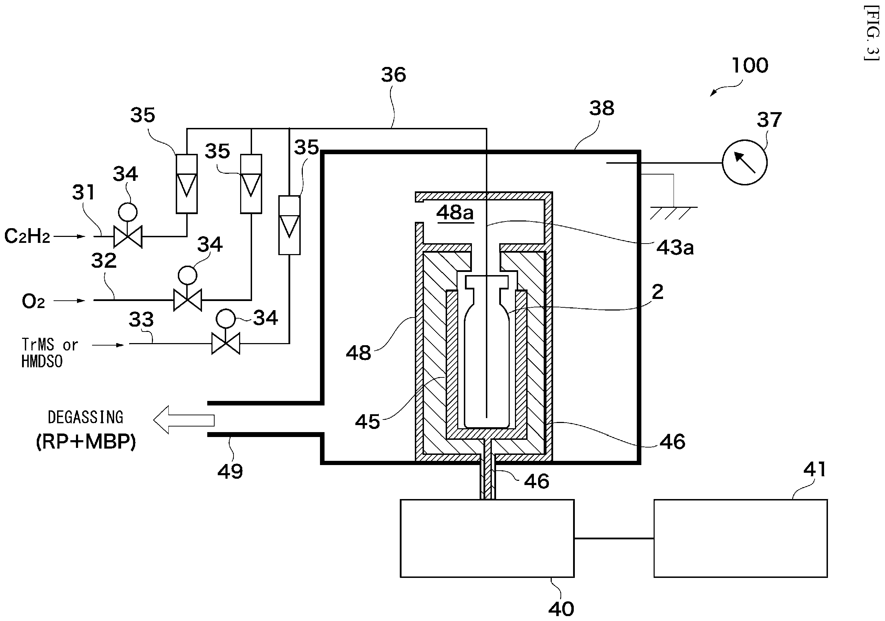

Next, before explaining the method for manufacturing the medical glass container, a manufacturing apparatus will be described. FIG. 3 is a schematic diagram illustrating a high frequency type inner surface film formation apparatus for a vial. The high frequency type inner surface film formation apparatus 100 for a vial illustrated in FIG. 3 has source gas inlet lines 31, 32, and 33. Each source gas inlet line has a stop valve 34 and a gas flowmeter 35, and is connected to a single mixed-gas pipe 36. Although FIG. 3 illustrates a configuration in which three source gas inlet lines are provided, more source gas inlet lines may also be provided. The pipe 36 is connected to a conductive pipe 43a which also serves as an internal electrode and a gas introduction pipe arranged in a vacuum chamber 38. The vacuum chamber 38 is grounded, and a vacuum gauge 37 is connected thereto. In addition, inside the vacuum chamber 38, a vial 2, an external electrode 45 arranged to surround a side surface and a bottom surface of the vial 2, a dielectric member 46 arranged to surround the external electrode 45, and an outer casing 48 that surrounds the dielectric member 46 and is formed of a conductive material for stabilizing plasma of the source gas are arranged. The vacuum chamber 38 is connected to a degassing pipe 49. In addition, the external electrode 45 is connected to an automatic matching device 40 so as not to be electrically connected to the vacuum chamber 38. The automatic matching device 40 is connected to a high frequency power supply 41. The high frequency is set to, for example, 1 to 100 MHz, or preferably 13.56 MHz. The source gas discharged from the conductive pipe 43a flows through the inside of the vial 2, is discharged from the tip opening thereof, passes through the space 48a provided in the upper side of the outer casing 48, and then reaches the internal space of the vacuum chamber 38. Then, the gas is discharged through the degassing pipe 49.

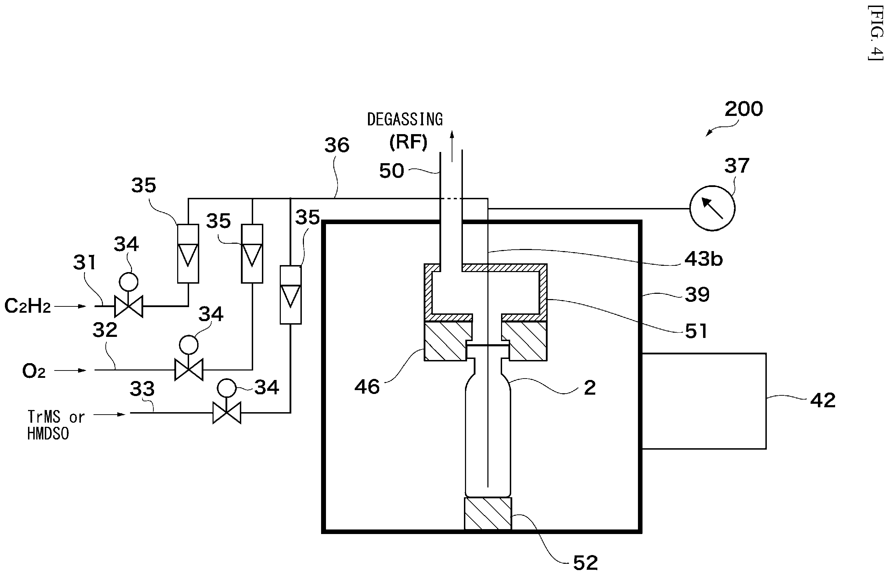

FIG. 4 is a schematic diagram illustrating a microwave type inner surface film formation apparatus for a vial. The microwave type inner surface film formation apparatus 200 for a vial illustrated in FIG. 4 has source gas inlet lines 31, 32, and 33. Each source gas inlet line has a stop valve 34 and a gas flowmeter 35, and is connected to a single mixed-gas pipe 36. Although FIG. 4 illustrates a configuration in which three source gas inlet lines are provided, more source gas inlet lines may also be provided. The pipe 36 is connected to a gas introduction pipe 43b arranged in the microwave shield 39. A vacuum gauge 37 is connected to the pipe 36. In addition, inside the microwave shield 39, a vial 2, a dielectric member 46 for fixing an opening of the vial 2 to prevent gas leakage, a pedestal 52 formed of a dielectric material for loading the vial 2, a vacuum room 51, and a degassing pipe 50 connected to the vacuum room 51 are arranged. The opening of the vial 2 is connected to the space inside the vacuum room 51. When the inside of the vacuum room 51 is evacuated, the internal space of the vial 2 is also evacuated. In addition, a microwave oscillator 42 for outputting a microwave is provided inside the microwave shield 39. The microwave frequency is, for example, 0.9 to 45 GHz, or preferably 2.45 GHz. The source gas discharged from the tip opening of the gas introduction pipe 43b flows through the inside of the vial 2, is discharged from its opening, passes through the inside of the vacuum room 51, and is then discharged through the degassing pipe 49.

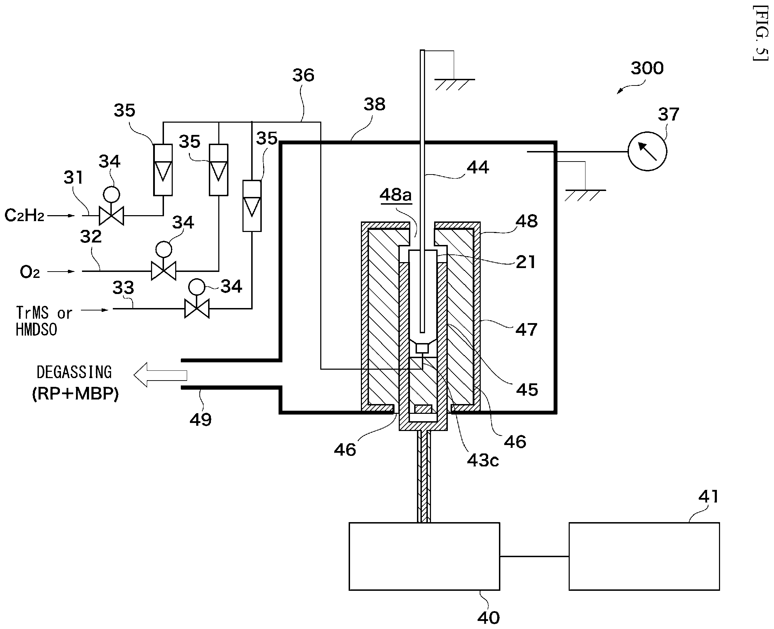

FIG. 5 is a schematic diagram illustrating a high frequency type inner surface film formation apparatus for an injector barrel. The high frequency type inner surface film formation apparatus 300 for an injector barrel illustrated in FIG. 5 has source gas inlet lines 31, 32, and 33. Each source gas inlet line has a stop valve 34 and a gas flowmeter 35, and is connected to a single mixed-gas pipe 36. Although FIG. 5 illustrates a configuration in which three source gas inlet lines are provided, more source gas inlet lines may also be provided. The pipe 36 is connected to a gas introduction pipe 43c arranged inside a vacuum chamber 38. The vacuum chamber 38 is grounded, and a vacuum gauge 37 is connected thereto. In addition, inside the vacuum chamber 38, an injector barrel 21, an external electrode 45 arranged to surround the injector barrel 21, a dielectric member 46 arranged to surround the external electrode 45, and an outer casing 48 arranged to surround the dielectric member 46 and formed of a conductive material for stabilizing plasma of the source gas are arranged. The vacuum chamber 38 is connected to the degassing pipe 49. In addition, the external electrode 45 is connected to an automatic matching device 40 so as not to be electrically connected to the vacuum chamber 38. The automatic matching device 40 is connected to a high frequency power supply 41. The frequency of the high frequency wave is, for example, 1 to 100 MHz, or preferably 13.56 MHz. A tip portion of the gas introduction pipe 43c is connected to a coupling portion which is the tip portion of the injector barrel 21. The source gas is delivered from the tip opening of the gas introducing pipe 43c to the coupling portion which is the tip portion of the injector barrel 21, flows inside the injector barrel 21, is discharged from the opening in the flange side of the injector barrel 21, passes through the space 48a provided in the upper side of the outer casing 48, and then reaches the internal space of the vacuum chamber 38. Then, the gas is discharged through the degassing pipe 49.

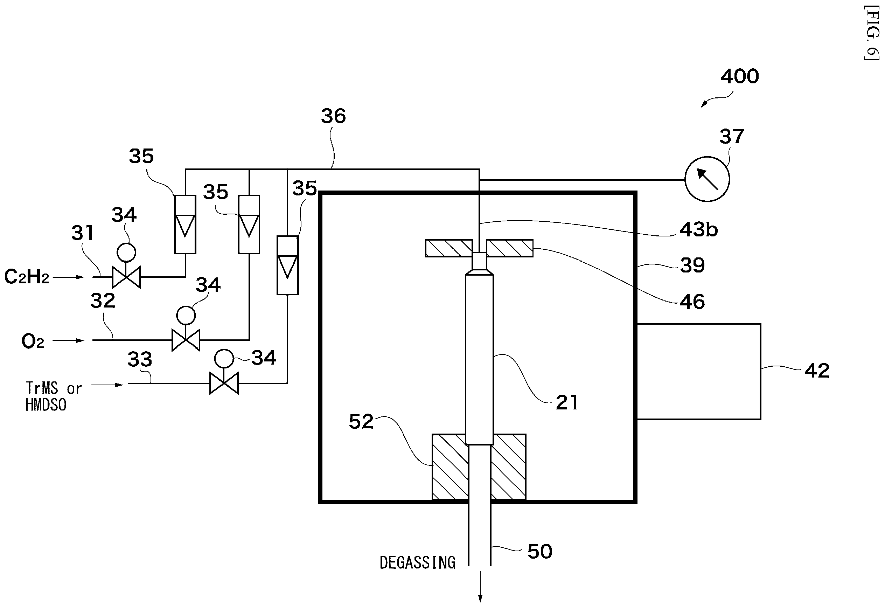

FIG. 6 is a schematic diagram illustrating a microwave type inner surface film formation apparatus for an injector barrel. The microwave type inner surface film formation apparatus 400 for an injector barrel illustrated in FIG. 6 has source gas inlet lines 31, 32, and 33. Each source gas inlet line has a stop valve 34 and a gas flowmeter 35, and is connected to a single mixed-gas pipe 36. Although FIG. 6 illustrates a configuration in which three source gas inlet lines are provided, more source gas inlet lines may also be provided. The pipe 36 is connected to a gas introduction pipe 43b arranged in the microwave shield 39. A vacuum gauge 37 is connected to the pipe 36. In addition, inside the microwave shield 39, an injector barrel 21, a dielectric member 46 for fixing a coupling portion (opening) of the tip portion of the injector barrel 21, a pedestal 52 formed of a dielectric material for loading a flange side of the injector barrel 21, and a degassing pipe 50 connected to the opening of the flange side of the injector barrel 21 are arranged. The coupling portion (opening) of the tip portion of the injector barrel 21 and the tip opening of the gas introduction pipe 43b are connected so as to prevent leakage. The opening of the flange side of the injector barrel 21 is connected to the degassing pipe 50. In addition, a microwave oscillator 42 for outputting a microwave is provided inside the microwave shield 39. The microwave frequency is, for example, 0.9 to 45 GHz, or preferably 2.45 GHz. The source gas introduced from the tip opening of the gas introduction pipe 43b flows through the inside of the injector barrel 21, is discharged from the opening of the flange side, and is then discharged through the degassing pipe 49.

(Method for Manufacturing Medical Glass Container)

A method for manufacturing a medical glass container according to the present embodiment is a method for manufacturing a medical glass container 1 or 20 having a coat 3 formed on at least a part of an inner wall of the medical glass container, the method including a process of forming a silicon-free diamond-like carbon film as the coat 3 by plasmatizing a silicon-free hydrocarbon-based gas as a constituent element inside a glass container, specifically inside a storage space of the vial 2 or the injector barrel 21.

Here, the silicon-free hydrocarbon-based gas includes a first hydrocarbon-based gas that does not contain fluorine or silicon as a constituent element (hereinafter, also referred to as "first hydrocarbon-based gas" in some cases), and a second hydrocarbon-based gas that is modified with fluorine and does not contain silicon as a constituent element (hereinafter, also referred to as "second hydrocarbon-based gas" in some cases).

The first hydrocarbon-based gas includes, for example, acetylene, methane, ethylene, or propane, and acetylene or methane is preferable.

The second hydrocarbon-based gas includes, for example, hexafluoroethane, $C_6F_{10}(CF_3)_2$, and $C_6F_6$, and hexafluoroethane is preferable.

In order to plasmatize the silicon-free hydrocarbon-based gas, for example, plasmatization is performed by applying a high frequency wave or a microwave using the film formation apparatuses illustrated in FIGS. 3 to 6.

According to the present embodiment, it is preferable that the silicon-free hydrocarbon-based gas is a mixed gas of the first hydrocarbon-based gas that does not contain fluorine or silicon as a constituent element and the second hydrocarbon-based gas that is modified with fluorine and does not contain silicon as a constituent element, and the coat is a silicon-free and fluorine-containing diamond-like carbon film. By using the aforementioned mixed gas, the fluorine content in the coat can be easily set to a desired ratio. The gas volume ratio of the mixed gas preferably satisfies a range of Formula 1. The fluorine content in the coat is within a predetermined range, for example, 8 to 50 atomic% (atomic percentage), and an excellent water repellency effect is obtained.

(Formula 1)

(second hydrocarbon-based gas):(first hydrocarbon-based gas) = 7:3 to 9:1

According to the present embodiment, it is preferable that the silicon-free hydrocarbon-based gas is the first hydrocarbon-based gas that does not contain fluorine or silicon as a constituent element, and the coat is a silicon-free and fluorine-free diamond-like carbon film. It is possible to easily form the silicon-free and fluorine-free diamond-like carbon film.

According to the present embodiment, it is preferable that, before forming the silicon-free diamond-like carbon film as a coat, a process of forming the intermediate layer on the glass surface of the inner wall of the vial 2 or the injector barrel 21 by plasmatizing the silicon-containing gas inside the storage space of the vial 2 or the injector barrel 21 is provided. By providing the silicon-containing intermediate layer, adhesion of the coat and uniformity of the coat are improved, and the inner surface temperature at the time of the hydrophilizing treatment increases, so that there is an effect of improving the density of the coat.

The silicon-containing gas includes, for example, a mixed gas containing an oxygen gas and trimethylsilane (TrMS), hexamethyldisiloxane (HMDSO), tetramethylorthosilicate ($Si(OCH_3)_4$), or tetraethoxysilane ($Si(OC_2H_5)_4$).

According to the present embodiment, when forming the coat, the mixing ratio between the silicon-containing gas and the silicon-free hydrocarbon-based gas may be set to 100:0 at the beginning of film formation, may be changed as the film formation time progresses, and may be set to 0:100 at the end of film formation. In this case, the coat becomes a gradient composition film.

According to the present embodiment, it is preferable that, before forming the coat and/or the intermediate layer, a hydrophilizing process for forming plasma by bring a hydrocarbon-based gas or an oxygen gas modified with fluorine into contact with at least a part of the inner surface of the vial 2 or the injector barrel 21 is provided. Since the glass surface of the inner wall of the vial 2 or the injector barrel 21 is hydrophilized, adhesion of the coat and uniformity of the coat are improved, and the inner surface temperature at the time of the hydrophilizing treatment increases, so that there is an effect of increasing the density of the coat. The hydrocarbon-based gas modified with fluorine includes hexafluoroethane, $C_6F_{10}(CF_3)_2$, and $C_6F_6$, and hexafluoroethane is preferable.

EXAMPLES

Hereinafter, the present invention will be described in more details with reference to examples, but the present invention is not construed as being limited to the examples.

Conditions for forming a film on a flat plate are as follows.

Equipment: Parallel plate low-pressure plasma CVD apparatus

High frequency output: 200 W, 13.56 MHz

Initial decompression: 0.02 torr

Film formation pressure: 0.1 torr

Film formation time: As shown in the following table

Mixed gas: As shown in the following table, where ratios indicate volume/flow-rate mixing ratio.

Pretreatment: None or $O_2$ plasma treatment for 5 min or $C_2F_6$ plasma treatment for 5 min

Base material: Borosilicate glass substrate (having a length of 20 mm, a width of 20 mm, and a thickness of 0.1 mm) (denoted as glass in the table) or Si substrate (having a length of 20 mm, a width of 20 mm, and a thickness of 0.38 mm) (denoted as "Si" in the table))

The evaluation was performed as follows.

Thickness: Step type thickness gauge (produced by DEKTAK XT, BRUKER)

Contact angle: The contact angle measurement device (DM300, produced by KYOWA) and pure water as an alternative to the aqueous content to be contacted were used. The measurement was performed by conforming to JIS R3257 "Testing method of wettability of glass substrate". Note that the aqueous content is a liquid chemical.

Adhesion: Cross-cut test, JIS K 5600-5-6 (1999)

The conditions for performing film formation on the inner surface of the container are as follows.

Equipment: Low pressure plasma CVD apparatus shown in FIG. 3 or 5

High frequency output: 100W, 13.56MHz

Initial decompression: 0.02 torr

Pressure for film formation: 2 torr

Film formation time: As shown in the following table

Mixed gas: As shown in the following table, where the ratios indicate volume/flow-rate mixing ratios

Pretreatment: None, $O_2$ plasma treatment for 5 min or $C_2F_6$ plasma treatment for 5 min

Base material: Borosilicate glass vial (having an outer diameter of 16 mm, an inner diameter of 14 mm, a height of 35 mm, and a volume of 2 ml) (in the table, referred to as "bottle", and hereinafter, also referred to as "vial" in some cases), or an injector barrel (having an outer diameter of 12.3 mm, an inner diameter of 10.6 mm, a length of 60 mm, and a volume of 2 ml) (in the table, referred to as "injector barrel")

(Example A)

An F-DLC film (hereinafter, referred to as "F-DLC" in the table) was deposited as a coat of the outermost layer. No intermediate layer was provided. The results are shown in Tables 1, 2, and 3.

[Table 1]

| Example A | Substrate | Outermost layer | Pretreatment | Gas | Film formation time (sec.) | Measured thickness (nm) | Contact angle (°) |
|---|---|---|---|---|---|---|---|
| 1 | glass | F-DLC | none | $C_2H_2$-$C_2F_6$ (2:8) | 20 | 56.8 | 96.7 |
| 2 | Si | F-DLC | none | $C_2H_2$-$C_2F_6$ (2:8) | 20 | 59.1 | 96.3 |
| 3 | glass | F-DLC | none | $C_2H_2$-$C_2F_6$ (6:4) | 6.25 | 14.6 | 77.1 |
| 4 | glass | F-DLC | none | $C_2H_2$-$C_2F_6$ (6:4) | 20 | 49.8 | 77.6 |
| 5 | glass | F-DLC | none | $C_2H_2$-$C_2F_6$ (4:6) | 6.25 | 16.4 | 80.1 |

[Table 2]

| Comparative example A | Substrate | Outermost layer | Pretreatment | Gas | Film formation time (sec.) | Measured thickness (nm) | Contact angle (°) |
|---|---|---|---|---|---|---|---|
| 1 | glass | glass | - | - | - | 0 | 30.5 |
| 2 | Si | Si | - | - | - | 0 | 55.0 |
| 3 | glass | F-DLC | none | $C_2H_2$-$C_2F_6$ (0:10) | 20 | 0 | 17.8 |

[Table 3]

| Comparative Example A' | Substrate | Outermost layer | Pretreatment | Gas | Film formation time (sec.) | Measured thickness (nm) | Contact angle (°) |
|---|---|---|---|---|---|---|---|
| 1 | glass | SiO:CH | none | TrMS-$O_2$(6:4) | 20 | 28.9 | 85.7 |

(Example B)

A DLC film (hereinafter, referred to as "DLC" in the table) was formed as a coat of the outermost layer. No intermediate layer was provided. The results are shown in Table 4.

[Table 4]

| Example B | Substrate | Outermost layer | Pretreatment | Gas | Film formation time (sec.) | Measured thickness (nm) | Contact angle (°) |
|---|---|---|---|---|---|---|---|
| 1 | glass | DLC | none | $C_2H_2$ | 2 | 3.8 | 80.8 |
| 2 | glass | DLC | none | C2H2 | 5 | 9.5 | 80.5 |

(Example C)

A DLC film was formed as a coat of the outermost layer. A silicon-containing intermediate layer (SiO:CH) was formed as the intermediate layer. The results are shown in Table 5.

[Table 5]

| Example C | Substrate | Outermost layer | Pretreatment | Gas (for outermost/ for intermediate layer) | Film formation time (sec.) (outermost/ intermediate layer) | Measured thickness (nm) (outermost/ intermediate | Contact angle (°) |
|---|---|---|---|---|---|---|---|
| 1 | glass | DLC | none | $C_2H_2$/TrMS-$O_2$(6:4) | 2/3 | 3.8/4.3 | 81.1 |
| 2 | glass | DLC | none | $C_2H_2$/TrMS-$O_2$(6:4) | 2/7 | 4.1/10.2 | 80.6 |
| 3 | glass | DLC | none | $C_2H_2$/TrMS-$O_2$(6:4) | 2/10 | 3.7/14.8 | 82.6 |

(Example D)

ADLC film or an F-DLC film was formed as a coat of the outermost layer. In Examples D-4 to D-6, a silicon-containing intermediate layer (SiO:CH) was formed as the intermediate layer. The results are shown in Table 6.

[Table 6]

| Example D | Substrate | Outermost layer | Pretreatment | Gas (uppermost layer) or (for outermost/ for intermediate layer) | Film formation time (sec.) (uppermost layer) or (for outermost/ for intermediate layer) | Measured thickness (nm) (uppermost layer) or (for outermost/ for intermediate layer) | Contact angle (°) |
|---|---|---|---|---|---|---|---|
| 1 | glass | DLC | none | $C_2H_2$ | 2 | 4.1 | 70.9 |
| 2 | glass | DLC | $O_2$ | $C_2H_2$ | 2 | 3.9 | 70.6 |
| 3 | glass | DLC | $C_2F_6$ | $C_2H_2$ | 2 | 4.0 | 81.6 |
| 4 | glass | DLC | none | $C_2H_2$/ TrMS-$O_2$ (6:4) | 2/2 | 3.8/3.1 | 77.4 |

(continued)

| Example D | Substrate | Outermost layer | Pretreatment | Gas (uppermost layer) or (for outermost/ for intermediate layer) | Film formation time (sec.) (uppermost layer) or (for outermost/ for intermediate layer) | Measured thickness (nm) (uppermost layer) or (for outermost/ for intermediate layer) | Contact angle (°) |
|---|---|---|---|---|---|---|---|
| 5 | glass | DLC | 02 | $C_2H_2$/ TrMS-$O_2$ (6:4) | 2/2 | 4.0/2.9 | 70.9 |
| 6 | glass | DLC | C2F6 | $C_2H_2$/ TrMS-$O_2$ (6:4) | 2/2 | 3.8/2.8 | 77.1 |

(Example E)

A DLC film or an F-DLC film was formed as a coat of the outermost layer for Examples E-1 and E-2. In Comparative example E-1, a glass substrate having no coat in the outermost layer was employed.

[Transmittance]

The measurement was performed in accordance with "JP17, 7.01 Test for Glass Containers for Injections, (5) Light transmission test for light-resistant container". Specifically, it was performed as follows. Absorption at a wavelength of 290 to 810 nm was measured using an ultraviolet/visible spectrophotometer (ASUV6300PC, produced by AS ONE corporation).

The measurement results for the contact angle and the transmittance are shown in Tables 7 and 8. In Tables 7 and 8, the transmittances at wavelengths of 290 nm, 450 nm, 590 nm, and 610 nm were compared.

[Table 7]

| Example E | Substrate | Outermost layer | Pretreatment | Gas (uppermost layer) | Film formation time (sec.) | Measured thickness (nm) | Contact angle (°) | Transmittance (%) 290nm/450nm/ 590nm/610nm |
|---|---|---|---|---|---|---|---|---|
| 1 | glass | DLC | none | $CH_4$ | 40 | 10.5 | 83.8 | 44.28/87.52/ 91.33/91.59 |
| 2 | glass | F-DLC | none | $CH_4/C_2F_6$(2:8) | 40 | 26.2 | 97.0 | 46.17/92.13/ 93.15/93.17 |

[Table 8]

| Comparative Example E | Substrate | Outermost layer | Pretreatment | Gas (uppermost layer) | Film formation time (sec.) | Measured thickness (nm) | Contact angle (°) | Transmittance (%) 290nm/ 450nm/ 590nm/610nm |
|---|---|---|---|---|---|---|---|---|
| 1 | glass | - | - | - | - | 0 | 32.0 | 52.50/92.45/ 93.30/93.32 |

(Example F)

A DLC film equivalent to that of Example E-1 or an F-DLC film equivalent to that of Example E-2 was formed on the inner wall of the medical glass container (vial having an outer diameter of 23 mm, an inner diameter of 21 mm, a height of 35 mm, and a volume of 5 ml, hereafter referred to as "vial 2") as a coat of the outermost layer in Examples F-1 and F-2. In Comparative example F-1, a medical glass container (vial 2) having no coat on the outermost layer was prepared.

[Remaining amount of water]

The remaining amount of water was measured as follows. Masses of the vial 2, the rubber stopper, and the aluminum cap were measured (mass 1). Then, the vial 2 is filled with ultra pure water at about 50% of the full volume, the rubber stopper and the aluminum cap were covered with lids, and then the mass was measured (mass 2). Then, the syringe was pierced into the rubber stopper, and the vial was sucked up while being turned upside down and was then weighed (mass 3). Each mass was measured using an electronic balance (MSA224S100DI, produced by SARTORIUS). The remaining amount of water was obtained by subtracting the mass 1 from the mass 3.

[Absorption amount of serum albumin]

First, the coated vial 2 was washed with ultra pure water and was dried at the room temperature. Then, a BSA (bovine serum albumin) solution of 10 ug/mL was transferred to each dried vial 2 by 1 mL, the vial 2 was moved to come into contact with the whole solution, and the solution was left at rest for 10 minutes. Then, the BSA solution in each vial 2 was taken by 10 ug, the fluorescence at a wavelength of 470/570 nm was measured, and the adsorption amount was calculated from the calibration curve. For the measurement of fluorescence, a plate reader (EnSpire, produced by Perkin Elmer, Inc.) was used.
Tables 9 and 10 show the measurement results for the remaining amount of water and the absorption amount of serum albumin.

[Table 9]

| Example F | Outermost layer | Remaining amount of water (g) | Absorption amount of serum albumin (ug/vial) |
|---|---|---|---|
| 1 | DLC | 0.009 | 3.65 |
| 2 | F-DLC | 0.004 | - |

[Table 10]

| Comparative example F | Outermost layer | Remaining amount of water (g) | Absorption amount of serum albumin (ug/vial) |
|---|---|---|---|
| 1 | - | 0.092 | 5.20 |

(Example G)

ADLC film equivalent to that of Example E-1 was formed on the inner wall of the medical glass container (vial 2) as a coat of the outermost layer in Example G-1. In Comparative example G, a medical glass container (vial 2) having no coat of the outermost layer was employed.

[Elution amount after steam sterilization]

Measurement was performed in accordance with "EP 8.4, 3.2.1. Glass containers for pharmaceutical use test A. hydrolytic resistance of the inner surface of glass containers (surface test)". Specifically, the measurement was performed as follows. Each vial 2 was filled with various solutions by 90% of the full volume to obtain an extract liquid subjected to the high-pressure stream sterilization at a temperature of 121°C for one hour. Then, a metal ion concentration of the extract liquid was measured using an ICP emission spectrometer (OPTIMA 8300, produced by PerkinElmer, Inc.).

[Number of insoluble particulate matter contained per 1 mL after steam sterilization]

The measurement was performed in accordance with "JP17, 6.07 Insoluble Particulate Matter Test for Injections, 1. Method 1. Light Obscuration Particle Count Test". Specifically, it was performed as follows. Each vial 2 was filled with various solutions by 90% of the full volume to obtain an extract liquid after the high-pressure steam sterilization at a temperature of 121°C for one hour. Then, the number of insoluble particulate matter in the extract liquid was measured using an in-liquid particle counter (KL-04, produced by RION Co., Ltd.).

Tables 11 and 12 show the measurement results of the elution amount after steam sterilization and the number of insoluble particulate matter contained per 1 mL after steam sterilization.

[Table 11]

| Example G | Elution amount after steam sterilization (mg/L) Si/Na | | | Number of insoluble particulate matter contained per 1 mL after steam sterilization (number) 1.5-2$\mu$m/2-3$\mu$m/10-25$\mu$m | | |
|---|---|---|---|---|---|---|
| | Water | KCl aqueous solution of 0.9wt% (pH=8) | Phthalic acid buffer solution (pH=4.01) | Water | KCl aqueous solution of 0.9wt% (pH=8) | Phthalic acid buffer solution (pH=4.01) |
| 1 | 0.164/ 0.031 | 0.214/ 1.999 | 0.165/ 0.63 | 13.5/4/0 | 12.5/5/1 | 32/8/0 |

[Table 12]

| Comparative Example G | Elution amount after steam sterilization (mg/L) Si/Na | | | Number of insoluble particulate matter contained per 1 mL after steam sterilization (number) 1.5-2$\mu$m/2-3$\mu$m/10-25$\mu$m | | |
|---|---|---|---|---|---|---|
| | Water | KCl aqueous solution of 0.9wt% (pH=8) | Phthalic acid buffer solution (pH=4.01) | Water | KCl aqueous solution of 0.9wt% (pH=8) | Phthalic acid buffer solution (pH=4.01) |
| 1 | 0.186/ 0.193 | 1.046/ 7.816 | 0.180/ 1.041 | 63.5/23/1 | 189/59.5/1.5 | 73.5/21.5/3.5 |

As shown in Tables 7 and 8, from comparison between Examples E-1 and E-2 and Comparative Example E-1, it is possible to increase the contact angle with the aqueous content by forming the diamond-like carbon film on the glass substrate. Furthermore, from comparison between Examples E-1 and E-2, it is possible to improve the transmittance of the glass substrate by incorporating a high content of fluorine into the diamond-like carbon film.

As shown in Tables 9 and 10, from comparison between Examples F-1 and F-2 and Comparative example F-1, it is possible to reduce the amount remaining in the container and suppress aggregation (absorption) of protein by forming the diamond-like carbon film on the inner wall of the medical glass container (vial). In addition, from comparison between Examples F-1 and F-2, it is possible to minimize the amount remaining in the container by incorporating a high content of fluorine into the diamond-like carbon film.

As shown in Tables 11 and 12, by forming a diamond-like carbon film corresponding to that of Example E-1 on the inner wall of a medical glass container (vial), it is possible to reduce the elution amount of metal ions (Si ions, Na ions) to the aqueous content and the amount of insoluble particulate matter and improve stability of the aqueous content.

DESCRIPTION OF REFERENCE SIGNS

| | |
|---|---|
| 1, 20 | medical glass container |
| 2 | glass container (vial as film formation target) |
| 2a | inner wall of vial |
| h | height direction of container |
| 3, 22 | coat |
| 4 | rubber stopper |

| 6, 25 | diamond-like carbon film |
|---|---|
| 21 | glass container (injector barrel as film formation target) |
| 21a | inner wall of injector barrel |
| 23 | plunger |
| 24 | gasket |
| 24a | surface of gasket |
| 26 | fluororesin film |
| 31, 32, 33 | source gas inlet line |
| 34 | stop valve |
| 35 | gas flowmeter |
| 36 | pipe |
| 37 | vacuum gauge |
| 38 | vacuum chamber |
| 39 | microwave shield |
| 40 | automatic matching device |
| 41 | high frequency power supply |
| 42 | microwave oscillator |
| 43a | conductive pipe |
| 43b | gas introduction pipe |
| 43c | gas introduction pipe |
| 45 | external electrode |
| 46 | dielectric member |
| 48 | outer casing |
| 48a | space |
| 49 | degassing pipe |
| 50 | degassing pipe |
| 51 | vacuum room |
| 52 | pedestal |
| 100 | high frequency type inner surface film formation apparatus for vial |
| 200 | microwave type inner surface film formation apparatus for vial |
| 300 | high frequency type inner surface film formation apparatus for injector barrel |
| 400 | microwave type inner surface film formation apparatus for injector barrel. |

## Claims

1. A medical glass container comprising a coat formed on at least a part of an inner wall of a glass container, wherein the coat is a silicon-free diamond-like carbon film.

2. The medical glass container according to claim 1, wherein the coat is a silicon-free and fluorine-containing diamond-like carbon film or a silicon-free and fluorine-free diamond-like carbon film.

3. The medical glass container according to claim 1 or 2, wherein a silicon-containing intermediate layer is provided between a glass surface of the inner wall of the glass container and the coat.

4. The medical glass container according to any one of claims 1 to 3, wherein at least a part of the glass surface of the inner wall of the glass container is hydrophilized.

5. The medical glass container according to any one of claims 1 to 4, wherein glass forming the glass container is borosilicate glass, aluminosilicate glass, or soda lime glass.

6. The medical glass container according to any one of claims 1 to 5, wherein the glass container is a vial, an injector barrel, a syringe with needle, and a cartridge type syringe.

7. The medical glass container according to claim 6, wherein the glass container is a vial,

the medical glass container further has a rubber stopper, and
at least a part of an inner surface of the rubber stopper is coated with a diamond-like carbon film or a fluororesin film.

8. The medical glass container according to claim 6, wherein
the glass container is an injector barrel,
the medical glass container further has a gasket-mounted plunger, and
at least a part of a surface of the gasket is coated with a diamond-like carbon film.

9. The medical glass container according to claim 6, wherein
the glass container is an injector barrel,
the medical glass container further has a gasket-mounted plunger, and
a fluororesin film is bonded to at least a face of a surface of the gasket adjoining with the inner surface of the injector barrel.

10. The medical glass container according to claim 2, wherein
the coat is a silicon-free and fluorine-containing diamond-like carbon film, and
a transmittance of the glass container at 450 nm is 90 % or higher.

11. A method for manufacturing a medical glass container having a coat formed on at least a part of an inner wall of the glass container, the method comprising
a process of forming a silicon-free diamond-like carbon film as the coat by plasmatizing a hydrocarbon-based gas that does not contain silicon as a constituent element inside a storage space of the glass container.

12. The method for manufacturing the medical glass container according to claim 11, wherein
the hydrocarbon-based gas that does not contain silicon is a mixed gas of a first hydrocarbon-based gas that does not contain fluorine or silicon as a constituent element and a second hydrocarbon-based gas that is modified with fluorine and does not contain silicon as a constituent element, and
the coat is a silicon-free/fluorine-containing diamond-like carbon film.

13. The method for manufacturing the medical glass container according to claim 11, wherein
the hydrocarbon-based gas that does not contain silicon is a first hydrocarbon-based gas that does not contain fluorine or silicon as a constituent element, and
the coat is a silicon-free/fluorine-free diamond-like carbon film.

14. The method for manufacturing the medical glass container according to claim 12 or 13, wherein
the first hydrocarbon-based gas is acetylene and/or methane.

15. The method for manufacturing the medical glass container according to claim 12, wherein
a gas volume ratio of the mixed gas satisfies a range of Formula 1,
where (Formula 1)
(second hydrocarbon-based gas):(first hydrocarbon-based gas) = 7:3 to 9:1.

16. The method for manufacturing the medical glass container according to any one of claims 11 to 15, wherein
the hydrocarbon-based gas that does not contain silicon is plasmatized by high frequency output or microwave output.

17. The method for manufacturing the medical glass container according to any one of claims 11 to 16, comprising
a process of forming an intermediate layer on a glass surface of the inner wall of the glass container by plasmatizing a silicon-containing gas inside a storage space of the glass container before forming the silicon-free diamond-like carbon film as the coat.

18. The method for manufacturing the medical glass container according to any one of claims 11 to 17, comprising
a hydrophilizing process for forming plasma by bringing a hydrocarbon-based gas or an oxygen gas modified with fluorine into contact with at least a part of an inner surface of the medical glass container before forming the coat and/or the intermediate layer.

[FIG.1]

[FIG.2]

[FIG. 3]

[FIG. 4]

[FIG. 5]

[FIG. 6]

## INTERNATIONAL SEARCH REPORT

| International application No. |
| --- |
| PCT/JP2018/045986 |

### A. CLASSIFICATION OF SUBJECT MATTER
Int.Cl. A61J1/05(2006.01)i

According to International Patent Classification (IPC) or to both national classification and IPC

### B. FIELDS SEARCHED

Minimum documentation searched (classification system followed by classification symbols)
Int.Cl. A61J1/05

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| Y | JP 2016-510288 A (CORNING INCORPORATED) 07 April 2016, paragraphs [0026]-[0027], [0050]-[0051], [0150], fig. 1, 5 & WO 2014/085242 A1, paragraphs [0083]-[0084], [0107]-[0108], [0206], fig. 1, 5 & US 2014/0151371 A1 & CA 2889763 A1 & KR 10-2015-0091369 A & CN 104968626 A | 1-18 |
| Y | JP 2012-036438 A (JTEKT CORP. et al.) 23 February 2012, paragraphs [0029]-[0031], [0046], [0109] & US 2012/0034393 A1, paragraphs [0041]-[0043], [0058], [0121] & EP 2415900 A1 & CN 102373437 A | 1-18 |

☒ Further documents are listed in the continuation of Box C.      ☐ See patent family annex.

| | |
| --- | --- |
| *    Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| 22 February 2019 (22.02.2019) | 05 March 2019 (05.03.2019) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2018/045986

C (Continuation).   DOCUMENTS CONSIDERED TO BE RELEVANT

| Category* | Citation of document, with indication, where appropriate, of the relevant passages | Relevant to claim No. |
|---|---|---|
| Y | US 4746538 A (CENTRE NATIONAL DE LA RECHERCHE SCIENTIFIQUE (CNRS)) 24 May 1988, column 1, lines 5-9, column 2, lines 7-19, column 2, line 63 to column 3, line 34 & JP 62-284081 A & EP 233825 A1 & FR 2592874 A1 | 1-10 |
| Y | WO 2016/132562 A1 (UTEC CORP.) 25 August 2016, abstract, page 3, lines 12-14, page 4, lines 33-37, page 6, lines 5-37, fig. 2 & TW 201634728 A | 2-10, 12-18 |
| Y | WO 2017/081409 A1 (APTAR STELMI SAS) 18 May 2017, page 7, line 29 to page 8, line 5, fig. 1 & US 2018/0319946 A1, paragraph [0050], fig. 1 & JP 2018-536738 A & FR 3043679 A1 & CN 108350205 A & KR 10-2018-0084084 A & TW 201716623 A | 7 |
| Y | JP 2001-190665 A (TERUMO CORP.) 17 July 2001, paragraph [0016], fig. 1 (Family: none) | 8 |
| Y | JP 2015-150377 A (SUMITOMO RUBBER INDUSTRIES, LTD.) 24 August 2015, paragraphs [0021]-[0022], fig. 1-2 & US 2015/0231337 A1, paragraphs [0036]-[0039], fig. 1-2 & EP 2910265 A1 & CN 104841043 A | 9 |
| Y | 高千穂化学工業株式会社, アセチレン, 安全データシート, 02 November 2015, non-official translation (TAKACHIHO CHEMICAL INDUSTRIAL CO., LTD., Acetylene, Safety data sheet) | 11-18 |
| A | US 2015/0158762 A1 (INTERMOLECULAR INC.) 11 June 2015, entire text, all drawings (Family: none) | 1, 11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000350770 A

- JP 2015180780 A

**Non-patent literature cited in the description**

- *Journal of Colloid and Interface Science,* September 1994, vol. 166 (2), 490-498

- Japanese Pharmacopoeia. 7